# EUROPÄISCHE PATENTSCHRIFT

(11) **EP 1 489 999 B1**
(45) Veröffentlichungstag und Bekanntmachung des Hinweises auf die Patenterteilung: **14.09.2005**
(21) Anmeldenummer: 03720173.8
(22) Anmeldetag: 13.03.2003
(51) Int. Cl.: A61F 2/42

(54) **KÜNSTLICHES GELENK**
ARTIFICIAL JOINT
ARTICULATION ARTIFICIELLE

(30) Priorität: 22.03.2002 DE 10213063
(43) Veröffentlichungstag der Anmeldung: 29.12.2004
(73) Patentinhaber: HJS Gelenk System GmbH, 81925 München (DE)
(72) Erfinder: NÄGERL, Hans, 37130 Gleichen (DE); THEUSNER, Joachim, 80539 München (DE); KUBEIN-MEESENBURG, Dietmar, 37075 Göttingen (DE)
(74) Vertreter: Scheffler, Jörg
(86) Internationale Anmeldenummer: PCT/DE2003/000827
(87) Internationale Veröffentlichungsnummer: WO 2003/079938

(56) Entgegenhaltungen:
- US-A- 3 839 742
- US-A- 3 886 599
- US-A- 3 975 778

## Beschreibung

Die Erfindung betrifft ein zum Ersatz eines oberen Sprunggelenkes bestimmtes künstliches Gelenk mit einer eine Gelenkpfanne bildenden, zum Ersatz der Tibia bestimmten ersten Hauptgelenkfläche, die aus zu einer der Sagittalebene entsprechenden Hauptfunktionsebene des Gelenkes parallelen, konkaven Kurvaturen aufgebaut ist, und mit einer mit der ersten Hauptgelenkfläche in Wirkverbindung tretenden zweiten Hauptgelenkfläche, als Bestandteil eines den Talus ersetzenden Gelenkkopfes mit in der Hauptfunktionsebene konvexen Kurvaturen, die auf die erste Hauptgelenkfläche abgestimmt sind, wobei die Radien der konvexen Kurvaturen der zweiten Hauptgelenkfläche kleiner als diejenigen der korrespondierenden Kurvaturen der ersten Hauptgelenkfläche bemessen sind.

Solche künstlichen Gelenke werden beispielsweise zum Ersatz des Sprunggelenkes in der Praxis vielfach eingesetzt und sind daher durch offenkundige Vorbenutzung bekannt. Als Grundform solcher Gelenke ist der Gelenkkopf beispielsweise kugel- oder zylinderförmig ausgeführt und ermöglicht dadurch einen oder mehrere Freiheitsgrade. Die korrespondierenden Gelenkflächen weisen dabei eine dem gewünschten Toleranzmaß entsprechende Differenz auf, die insbesondere den Anforderungen an eine hohe Belastbarkeit bei zugleich geringem mechanischem Verschleiß genügen muss.

In der medizinischen Praxis hat es sich jedoch gezeigt, dass sich die Eigenschaften natürlicher Gelenke mit Hilfe der bekannten künstlichen Gelenke nur sehr unvollkommen nachbilden lassen. Insbesondere können die Eigenschaften natürlicher Gelenke, die bei Belastung zur Übertragung hoher Kräfte geeignet sind, während sie bei verminderter Belastung zugleich ein hohes Maß an Beweglichkeit aufweisen, nicht durch künstliche Gelenke realisiert werden. Dies führt dazu, dass oftmals auf die an sich wünschenswerte Beweglichkeit zugunsten erhöhter Dauerbelastbarkeit verzichtet werden muss.

Der Erfindung liegt daher die Aufgabe zugrunde, eine Möglichkeit zu schaffen, die Beweglichkeit des künstlichen Gelenkes bei einer zugleich hohen Dauerbelastbarkeit zu realisieren. Insbesondere soll dabei eine Verringerung der Dauerbelastbarkeit aufgrund der erhöhten Bewegungsfreiheit vermeidbar sein.

Diese Aufgabe wird erfindungsgemäß mit einem künstlichen Gelenk gemäß den Merkmalen des Patentanspruchs 1 gelöst. Die Unteransprüche betreffen besonders zweckmäßige Weiterbildungen der Erfindung.

Erfindungsgemäß ist also ein künstliches Gelenk vorgesehen, bei dem die Radien derart bemessen sind, dass die auftretenden Differenzbeträge der korrespondierenden Radien der ersten Hauptgelenkfläche und der zweiten Hauptgelenkfläche in einer nach vom weisenden und einer nach hinten weisenden Winkelstellung des Gelenkes voneinander abweichen. Aufgrund der abweichenden Differenzbeträge zwischen der nach vorn weisenden und einer nach hinten weisenden Winkelstellung des Gelenkes wird ein zusätzlicher Freiheitsgrad in Abhängigkeit der Winkelstellung realisiert, der ohne eine Einschränkung der Belastbarkeit des Gelenkes in anderen Winkelstellungen eine verbesserte Beweglichkeit ermöglicht. Dabei wird es beispielsweise erstmals möglich, dass der Gelenkkopf in einer mit hohen Differenzbeträgen verbundenen Winkelstellung einerseits um eine im Gelenkkopf zentrierte Achse schwenkbar und zusätzlich um eine durch die Kontaktfläche zwischen dem Gelenkkopf und der Gelenkpfanne definierte Achse schwenkbar ist, wodurch ein zusätzlicher Freiheitsgrad erreicht wird. Die körperbezogene Lage der unterschiedlichen Differenzbeträge ist dabei von der gewünschten Belastbarkeit abhängig und insbesondere bei einer statischen Belastung, beispielsweise im Stehen, geringer als bei einer dynamischen Belastung.

Eine besonders vorteilhafte Ausführungsform der vorliegenden Erfindung wird auch dadurch erreicht, dass die Radien der konvexen Kurvaturen der zweiten Hauptgelenkfläche 5 - 20 % kleiner als diejenigen der korrespondierenden Kurvaturen der ersten Hauptgelenkfläche bemessen sind. Hierdurch wird ein in der Praxis optimales Verhältnis zwischen der gewünschten Beweglichkeit einerseits und der Belastbarkeit andererseits erreicht, was in der Praxis näherungsweise einem Differenzmaß zwischen 1 und 5 mm entspricht.

Die Formgebung der Hauptgelenkfläche könnte über die gesamte Breite senkrecht zu der Hauptfunktionsebene gleich bleibend ausgeführt sein. Als besonders Erfolg versprechend erweist sich jedoch in der Praxis eine Abwandlung, bei der die Differenzbeträge der Radien zwischen einer der Körpermitte zugewandten medialen Seite und einer dem Körperäußeren zugewandten lateralen Seite bei übereinstimmenden Winkelstellungen des Gelenkes voneinander abweichen. Hierdurch wird die Ausbildung abweichender Kontaktflächen zwischen der medialen und der lateralen Seite begünstigt, was insbesondere im Bereich geringerer Differenzbeträge zu einer vergleichsweise großflächigen Kontaktfläche führt, während sich die Kontaktfläche auf der gegenüberliegenden Seite einer Kontaktlinie annähert und dadurch die Beweglichkeit begünstigt. Dadurch können die dem menschlichen Bewegungsablauf entsprechenden Gelenkeigenschaften umgesetzt werden. Ferner kann auch eine Beweglichkeit quer zu der Hauptfunktionsebene problemlos umgesetzt werden.

Eine weitere besonders praxisnahe Weiterbildung der vorliegenden Erfindung wird auch dadurch erreicht, dass die Kurvaturen paralleler Ebenen der zweiten Hauptgelenkfläche in der nach vom bzw. oben weisenden Winkelstellung der lateralen Seite zu der medialen Seite des Gelenkes und zugleich die Kurvaturen paralleler Ebenen der zweiten Hauptgelenkfläche in der nach hinten bzw. unten weisenden Winkelstellung der medialen Seite zu der lateralen Seite des Gelenkes abnehmende Radien aufweisen. Hierdurch tritt eine Umkehrung der Ausprägung der Kontaktfläche zwischen der ersten und der zweiten Hauptgelenkfläche, bezogen auf die Ebene quer zu der Hauptfunktionsebene, in Abhängigkeit von der Winkelstellung des Gelenkes ein. Auf diese Weise können die gewünschten Freiheitsgrade jeder individuellen Winkelstellung entsprechend den erforderlichen Eigenschaften angepasst werden. Die beiden Hauptgelenkflächen berühren sich in einem linienförmigen Kontakt, der ungefähr senkrecht zur Hauptfunktionsebene angeordnet ist. Aufgrund der variierenden Radien in der Hauptgelenkfläche ist unter Kraftschluss der linienförmige Kontakt zwischen den Hauptgelenkflächen in der nach vom weisenden Winkelstellung keilförmig mit der Spitze des Keiles auf der medialen Seite und in der nach hinten weisenden Winkelstellung mit der Spitze des Keiles lateralen Seite zu finden. Der Grund der keilförmigen Kontaktlinie liegt in der differierenden Inkongruenz.

Dabei erweist sich in der Praxis eine Abwandlung des Gelenkes als besonders gut geeignet, wenn die Kurvaturen aus wenigen Einzelradien mit einem stetigen Übergang aufgebaut sind. Hierdurch kann der mechanische Verschleiß auf ein Minimum beschränkt und zugleich ein für den Patienten harmonischer Bewegungsablauf realisiert werden.

Eine andere besonders vorteilhafte Ausgestaltung wird auch dann erreicht, wenn die erste Hauptgelenkfläche oder die zweite Hauptgelenkfläche quer zu der Hauptfunktionsebene in der vorderen Stellung breiter als in der hinteren Stellung ausgeführt ist. Aufgrund dieser keilsegmentförmigen ersten und zweiten Hauptgelenkfläche sowie insbesondere auch der angeschlossenen Seitenflächen, hat das Gelenk bei einem vom liegenden Kontakt unter Kraftschluss auf die erste Hauptgelenkfläche und zugleich hinten liegendem Gegenkontakt auf der zweiten Hauptgelenkfläche nur zwei Freiheitsgrade. Bei hinten liegendem Kontakt unter Kraftschluss auf der ersten Hauptgelenkfläche und vorne liegendem Gegenkontakt auf der zweiten Hauptgelenkfläche hat das Gelenk vier und bei massivem Kraftschluss drei Freiheitsgrade.

Hierzu eignet sich in der Praxis eine Ausgestaltung, bei der das Größenverhältnis der ersten Hauptgelenkfläche gegenüber der zweiten Hauptgelenkfläche näherungsweise 2:3 beträgt, um so ein Optimum der erreichbaren Freiheitsgrade ohne signifikante Einschränkung der Belastbarkeit zu erreichen.

In der Praxis erweist sich dabei eine Ausführungsform der Erfindung als besonders günstig, wenn die erste Hauptgelenkfläche oder die zweite Hauptgelenkfläche einem Oberflächenausschnitt eines rotationssymmetrischen Körpers, insbesondere eines Zylinders, eines Kegels oder eines Rotationshyperboloides entspricht, um so je nach den auftretenden Kräfteverhältnissen eine resultierende Krafteinleitung zu erreichen, deren vektorielle Komponenten eine Verstärkung der vorhandenen anatomischen Gegebenheiten ermöglicht. Dabei ermöglicht insbesondere ein Übergang von einer Konvexität zu einer Konkavität zwischen der medialen und der lateralen Seite eine Umkehrung der Kontaktfläche zwischen den entgegen gesetzten Gelenkstellungen und damit der Lastverteilung.

Überraschenderweise werden die Eigenschaften des Gelenkes dadurch weiter verbessert, dass die zweite Hauptgelenkfläche auf der medialen Seite quer zu der Hauptfunktionsebene eine Konvexität und wahlweise zusätzlich die zweite Hauptgelenkfläche auf der lateralen Seite quer zu der Hauptfunktionsebene über eine Konvexität in eine der Hauptfunktionsebene angenäherte Konkavität übergeht, wobei die korrespondierende erste Hauptgelenkfläche eine daran angepasste Formgebung mit einem geringfügigen Differenzmaß aufweist. Die erste Hauptgelenkfläche weist dabei entsprechende umgekehrte, zugeordnete Krümmungen auf, deren Konkavität gegebenenfalls übereinstimmende oder geringfügig größer bemessene Radien und deren Konvexität gegebenenfalls übereinstimmende oder geringfügig kleiner bemessene Radien aufweisen. Die zweite Hauptgelenkfläche fällt zur medialen Seite über einen konvexen Übergang in einer Konkavität steil ab, um dann in einem lateralen Pol zu enden.

Die zweite Hauptgelenkfläche dient dem abschnittsweisen Ersatz des Talus. Besonders zweckmäßig ist es dabei auch, wenn die erste Hauptgelenkfläche als eine Einheit zugleich der Tibia und der Fibula zugeordnet ist. Hierdurch kann die Einheit mit den vorhandenen Strukturen mit geringem operativen Aufwand verbunden werden, wobei die relative Stellung durch die Einheit vorgegeben ist.

Hingegen ermöglicht eine weitere besonders Erfolg versprechende Abwandlung der Erfindung, bei der die erste Hauptgelenkfläche aus zwei Strukturelementen besteht, einen unabhängigen Austausch lediglich einzelner, geschädigter Gelenkteile. Diese Strukturelemente können hierzu beispielsweise elastisch verbunden sein und dadurch eine eingeschränkte relative Beweglichkeit, insbesondere bei Überschreiten einer maximalen Belastung, aufweisen. Die Strukturelemente werden hierzu mit der Knochenstruktur einzeln verbunden.

Hierzu eignet sich in besonderer Weise eine Ausgestaltung, bei der die beiden Strukturelemente in einer zu der Hauptfunktionsebene parallelen Ebene miteinander verbunden sind, um so eine einfache Trennungsebene realisieren zu können. Jeweils eines der Strukturelemente kann dann mit der Tibia und der Fibula separat verbunden werden.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt jeweils in einer perspektivischen Darstellung in
- Fig.1: eine erste Hauptgelenkfläche eines erfindungsgemäßen Gelenkes;
- Fig.2: eine zweite Hauptgelenkfläche des Gelenkes.

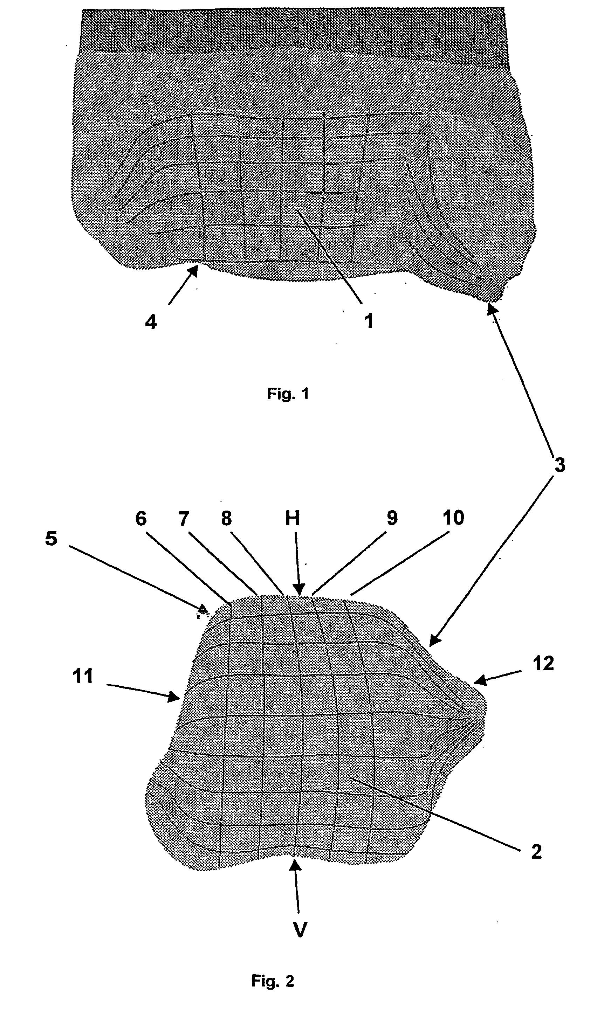

Die Figuren 1 und 2 zeigen jeweils eine erste Hauptgelenkfläche 1 und eine zweite Hauptgelenkfläche 2 eines insbesondere zum Ersatz eines oberen Sprunggelenkes bestimmten künstlichen Gelenkes 3, deren Zusammenwirken nachstehend mit Bezug auf beide Figuren näher erläutert wird. Die erste Hauptgelenkfläche 1 bildet durch ihre weitgehend konkave Formgebung eine Gelenkpfanne 4 und ist daher beispielsweise zum Ersatz der Tibia bestimmt, während eine zweite Hauptgelenkfläche 2 als Bestandteil eines insbesondere den Talus ersetzenden Gelenkkopfes 5 mit weitgehend konvexer Formgebung eine Beweglichkeit mit vorbestimmten Freiheitsgraden ermöglicht. Die auf diese Weise vornehmlich erreichbare Beweglichkeit entspricht einer zu der Zeichnungsebene senkrechte, der Sagittalebene entsprechenden Hauptfunktionsebene des Gelenkes 3. Die dabei wirkenden Kurvaturen 6, 7, 8, 9, 10 der zweiten Hauptgelenkfläche 2 sind dabei in Richtung von einer der Körpermitte zugewandten medialen Seite 11 hin zu einer dem Körperäußeren zugewandten lateralen Seite 12 aufsteigend beziffert. Die auftretenden Differenzbeträge der korrespondierenden Radien der Kurvaturen 6, 7, 8, 9, 10 der zweiten Hauptgelenkfläche 2 gegenüber der ersten Hauptgelenkfläche 1 sind dabei derart bemessen, dass deren Differenz in einer jeweiligen, nach vorn bzw. nach oben weisenden Winkelstellung V und einer nach unten weisenden Winkelstellung H des Gelenkes 3 voneinander zwischen 5 und 20 % differiert. Zugleich weisen die Kurvaturen 6, 7, 8, 9, 10 in der nach oben weisenden Winkelstellung V der medialen Seite 11 gegenüber der nach oben weisenden Winkelstellung V der lateralen Seite 12 des Gelenkes 3 einen zunehmenden Radius auf, während die Kurvaturen 6, 7, 8, 9, 10 in der nach hinten bzw. nach unten weisenden Winkelstellung H der medialen Seite 11 gegenüber der nach unten weisenden Winkelstellung H der lateralen Seite 12 des Gelenkes 3 einen abnehmenden Radius aufweisen.

Die erste Hauptgelenkfläche 1 und die zweite Hauptgelenkfläche 2 berühren sich dadurch entlang einer linienförmigen Kontaktfläche, die annähernd senkrecht zur Hauptfunktionsebene des Gelenkes 3 verläuft. Aufgrund der variierenden Radien der Kurvaturen 6, 7, 8, 9, 10 der zweiten Hauptgelenkfläche 2 ist unter Kraftschluss der linienförmige Kontakt auf der ersten Hauptgelenkfläche 1 in der nach oben weisenden Winkelstellung V keilförmig mit der Spitze des Keiles zur medialen Seite 11 und in der nach unten weisenden Winkelstellung H mit der Spitze des Keiles zur lateralen Seite 12 zu finden. Der Grund der keilförmigen Kontaktlinie liegt in der differierenden Inkongruenz. Hierdurch wird eine zugleich hohe Belastbarkeit und optimale Beweglichkeit des Gelenkes 3 in Abhängigkeit von der jeweiligen Gelenkstellung erreicht.

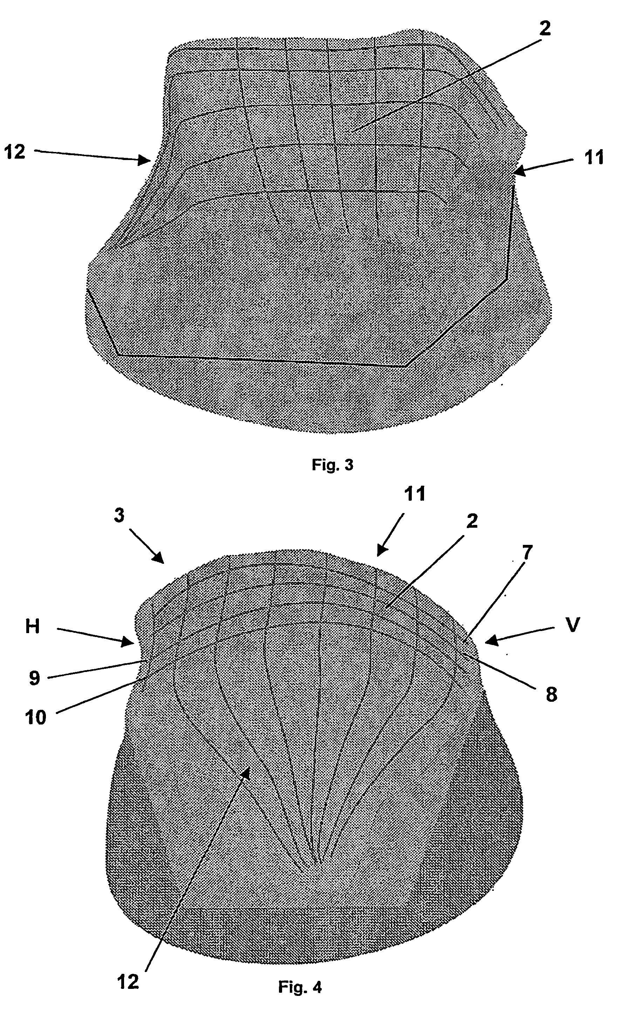

Figur 3 zeigt eine Draufsicht auf die zweite Hauptgelenkfläche 2, in der insbesondere der konvexe Übergang von der lateralen Seite 12 über eine Konkavität in einer Konvexität der medialen Seite 11 zu erkennen ist, in dessen weiterem Verlauf die zweite Hauptgelenkfläche 2 über eine Konkavität steil abfällt und in einem lateralen Pol endet.

Figur 4 zeigt die zweite Hauptgelenkfläche 2 mit ihren Kurvaturen aus einer Ansicht senkrecht zu der Hauptfunktionsebene. Zu erkennen sind die Radien der Kurvaturen 9, 10, die auf der lateralen Seite 12 in der nach oben weisenden Winkelstellung V kleiner als in der nach unten weisenden Winkelstellung H ausgeführt sind, während die Radien der Kurvaturen 7, 8 und auf der medialen Seite 11 in der nach oben weisenden Winkelstellung V größer als in der nach unten weisenden Winkelstellung H bemessen sind. Zugleich nehmen die Radien der Kurvaturen 7, 8, 9, 10 paralleler Ebenen der zweiten Hauptgelenkfläche 2 in der nach oben weisenden Winkelstellung V von der lateralen Seite 12 hin zu der medialen Seite 11 des Gelenkes 3 ab, wobei umgekehrt die Kurvaturen 7,8,9,10 in der nach unten weisenden Winkelstellung H zunehmende Radien aufweisen.

## Patentansprüche

1. Ein zum Ersatz eines oberen Sprunggelenkes bestimmtes künstliches Gelenk mit einer eine Gelenkpfanne bildenden, zum Ersatz der Tibia bestimmten ersten Hauptgelenkfläche, die aus einer zu einer der Sagittalebene entsprechenden Hauptfunktionsebene des Gelenkes parallelen, konkaven Kurvaturen aufgebaut ist, und mit einer mit der ersten Hauptgelenkfläche in Wirkverbindung tretenden zweiten Hauptgelenkfläche, als Bestandteil eines den Talus ersetzenden Gelenkkopfes mit in der Hauptfunktionsebene konvexen Kurvaturen, die auf die erste Hauptgelenkfläche abgestimmt sind, wobei die Radien der konvexen Kurvaturen der zweiten Hauptgelenkfläche kleiner als diejenigen der korrespondierenden Kurvaturen der ersten Hauptgelenkfläche bemessen sind, **dadurch gekennzeichnet, dass** die auftretenden Differenzbeträge der korrespondierenden Radien der ersten Hauptgelenkfläche (1) und der zweiten Hauptgelenkfläche (2) in einer nach oben weisenden Winkelstellung (V) und einer nach unten weisenden Winkelstellung (H) des Gelenkes (3) voneinander abweichen.

2. Gelenk nach Anspruch 1, **dadurch gekennzeichnet, dass** die Radien der konvexen Kurvaturen (7, 8, 9, 10) der zweiten Hauptgelenkfläche (2) 5 - 20 % kleiner als diejenigen der korrespondierenden Kurvaturen der ersten Hauptgelenkfläche (1) bemessen sind.

3. Gelenk nach den Ansprüchen 1 oder 2, **dadurch gekennzeichnet, dass** die Differenzbeträge der Radien zwischen einer der Körpermitte zugewandten medialen Seite (11) und einer dem Körperäußeren zugewandten lateralen Seite (12) bei übereinstimmenden Winkelstellungen (V, H) des Gelenkes (3) voneinander abweichen.

4. Gelenk nach zumindest einem der vorhergehenden Ansprüche, **dadurch gekennzeichnet, dass** die Kurvaturen (7, 8, 9, 10) paralleler Ebenen der zweiten Hauptgelenkfläche (2) in der nach oben weisenden Winkelstellung (V) der lateralen Seite (12) zu der medialen Seite (11) des Gelenkes (3) abnehmende Radien aufweisen.

5. Gelenk nach zumindest einem der vorhergehenden Ansprüche, **dadurch gekennzeichnet, dass** die Kurvaturen (7, 8, 9, 10) paralleler Ebenen der zweiten Hauptgelenkfläche (2) in der nach unten weisenden Winkelstellung (H) der medialen Seite (11) zu der lateralen Seite (12) des Gelenkes (3) abnehmende Radien aufweisen.

6. Gelenk nach zumindest einem der vorhergehenden Ansprüche, **dadurch gekennzeichnet, dass** die Radien der Kurvaturen (7, 8, 9, 10) auf der lateralen Seite (12) in der nach oben weisenden Winkelstellung (V) kleiner als in der nach unten weisenden Winkelstellung (H) und auf der medialen Seite (11) in der nach oben weisenden Winkelstellung (V) größer als in der nach unten weisenden Winkelstellung (H) bemessen sind.

7. Gelenk nach zumindest einem der vorhergehenden Ansprüche, **dadurch gekennzeichnet, dass** die Kurvaturen (7, 8, 9, 10) aus wenigen Einzelradien mit einem stetigen Übergang aufgebaut sind.

8. Gelenk nach zumindest einem der vorhergehenden Ansprüche, **dadurch gekennzeichnet, dass** die erste Hauptgelenkfläche (1) und / oder die zweite Hauptgelenkfläche (2) quer zu der Hauptfunktionsebene in der nach vom weisenden Winkelstellung (V) breiter als in der nach unten weisenden Winkelstellung (H) ausgeführt ist.

9. Gelenk nach zumindest einem der vorhergehenden Ansprüche, **dadurch gekennzeichnet, dass** das Größenverhältnis der ersten Hauptgelenkfläche (1) gegenüber der zweiten Hauptgelenkfläche (2) näherungsweise 2:3 beträgt.

10. Gelenk nach zumindest einem der vorhergehenden Ansprüche, **dadurch gekennzeichnet, dass** die erste Hauptgelenkfläche (1) und / oder die zweite Hauptgelenkfläche (2) einem Oberflächenausschnitt eines rotationssymmetrischen Körpers entspricht.

11. Gelenk nach Anspruch 10, **dadurch gekennzeichnet, dass** der Oberflächenausschnitt einem Zylinder oder einem Kegel entspricht.

12. Gelenk nach Anspruch 10, **dadurch gekennzeichnet, dass** der Oberflächenausschnitt einem Rotationshyperboloid entspricht.

13. Gelenk nach Anspruch 10, **dadurch gekennzeichnet, dass** der Oberflächenausschnitt von der Konvexität der medialen Seite (11) zu einer Konkavität auf der zweiten Hauptgelenkfläche (2) über eine Konvexität der lateralen Seite (12) in eine steil abfallende Konkavität übergeht, die in einen lateralen Pol mündet.

14. Gelenk nach zumindest einem der vorhergehenden Ansprüche, **dadurch gekennzeichnet, dass** die korrespondierende erste Hauptgelenkfläche (1) eine an die zweite Hauptgelenkfläche (2) angepasste Formgebung mit einem geringfügigen Differenzmaß aufweist.

15. Gelenk nach zumindest einem der vorhergehenden Ansprüche, **dadurch gekennzeichnet, dass** die erste Hauptgelenkfläche (1) als eine Einheit zugleich der Tibia und der Fibula zugeordnet ist.

16. Gelenk nach zumindest einem der vorhergehenden Ansprüche, **dadurch gekennzeichnet, dass** die erste Hauptgelenkfläche (1) aus zwei Strukturelementen besteht.

17. Gelenk nach Anspruch 16, **dadurch gekennzeichnet, dass** die beiden Strukturelemente in einer zu der Hauptfunktionsebene parallelen Ebene miteinander verbunden sind.

## Claims

1. Artificial joint intended to replace an upper ankle joint, comprising a first primary joint surface, which forms an articular socket, is intended to replace the tibia and is constructed from concave curvatures that run parallel to a primary functional plane, corresponding to the sagittal plane, of the joint, and comprising a second primary joint surface that cooperates with the first primary joint surface as a component of a condyle that replaces the talus, with convex curvatures, which are adapted to the first primary joint surface, in the primary functional plane, the radii of the convex curvatures of the second primary joint surface being smaller than those of the corresponding curvatures of the first primary joint surface, **characterized in that** the differential amounts that occur in the corresponding radii of the first primary joint surface (1) and the second primary joint surface (2) deviate from one another in an upwardly pointing angular position (V) and a downwardly pointing angular position (H) of the joint (3).

2. Joint according to claim 1, **characterized in that** the radii of the convex curvatures (7, 8, 9, 10) of the second primary joint surface (2) are 5 to 20% smaller than those of the corresponding curvatures of the first primary joint surface (1).

3. Joint according to claim 1 or claim 2, **characterized in that** the differential amounts of the radii between a medial side (11) facing the body centre and a lateral side (12) facing the body exterior deviate from one another when the angular positions (V, H) of the joint (3) correspond.

4. Joint according to at least one of the preceding claims, **characterized in that**, in the upwardly pointing angular position (V) of the lateral side (12) to the medial side (11) of the joint (3), the curvatures (7, 8, 9, 10) of parallel planes of the second primary joint surface (2) exhibit decreasing radii.

5. Joint according to at least one of the preceding claims, **characterized in that**, in the downwardly pointing angular position (H) of the medial side (11) to the lateral side (12) of the joint (3), the curvatures (7, 8, 9, 10) of parallel planes of the second primary joint surface (2) exhibit decreasing radii.

6. Joint according to at least one of the preceding claims, **characterized in that**, on the lateral side (12), the radii of the curvatures (7, 8, 9, 10) are smaller in the upwardly pointing angular position (V) than in the downwardly pointing angular position (H) and, on the medial side (11), are greater in the upwardly pointing angular position (V) than in the downwardly pointing angular position (H).

7. Joint according to at least one of the preceding claims, **characterized in that** the curvatures (7, 8, 9,10) are formed from a small number of individual radii having a constant transition.

8. Joint according to at least one of the preceding claims, **characterized in that**, transversely to the primary functional plane, the first primary joint surface (1) and/or the second primary joint surface (2) is wider in the forwardly pointing angular position (V) than in the downwardly pointing angular position (H).

9. Joint according to at least one of the preceding claims, **characterized in that** the size ratio of the first primary joint surface (1) to the second primary joint surface (2) is approximately 2:3.

10. Joint according to at least one of the preceding claims, **characterized in that** the first primary joint surface (1) and/or the second primary joint surface (2) corresponds to a surface section of a rotationally symmetrical body.

11. Joint according to claim 10, **characterized in that** the surface section corresponds to a cylindcr or a cone.

12. Joint according to claim 10, **characterized in that** the surface section corresponds to a rotational hyperboloid.

13. , Joint according to claim 10, ebaractcnzed in that the surface section changes from the convexity of the medial side (11) to a consocket on the second primary joint surface (2), via a convexity of the lateral side (12), into a steeply descending consocket, which merges into a lateral pole.

14. , Joint according to at least one of the preceding claims, **characterized in that** the corresponding first primary joint surface (1) has a form that is adapted to the second primary joint surface (2), with a small degree of difference.

15. Joint according to at least one of the preceding claims, **characterized in that** the first primary joint surface (1), as a unit, is associated with both the tibia and the fibula.

16. Joint according to at least one of the preceding claims, **characterized in that** the first primary joint surface (1) consists of two structural elements.

17. Joint according to claim 16, **characterized in that** the two structural elements are connected to each other in a plane parallel to the primary functional plane.

## Revendications

1. Articulation artificielle destinée à remplacer une articulation tibio-tarsienne supérieure, comportant une première surface d'articulation principale formant une cavité cotyloïde pour le remplacement du tibia et qui est constituée de courbures concaves parallèles à un plan fonctionnel principal de l'articulation correspondant au plan sagital, et comportant une deuxième surface d'articulation principale entrant en interaction avec la première surface d'articulation principale en tant qu'élément constituant une tête d'articulation remplaçant l'astragale, comportant dans le plan fonctionnel principal des courbures convexes qui sont adaptées à la courbure concave de la première surface d'artieulation principale, les rayons des courbures convexes de la deuxième surface d'articulation principale étant dimensionnés pour être plus petits que ceux de la courbure correspondante de la première surface d'articulation principale, **caractérisée en ce que** les différences de valeur entre les rayons respectifs de la première surface d'articulation principale (1) et de la deuxième surface d'articulation principale (2) diffèrent l'une de l'autre dans une position angulaire orientée vers le haut (V) et dans une position angulaire orientée vers le bas (H) de l'articulation (3).

2. Articulation selon la revendication 1, **caractérisée en ce que** les rayons des courbures convexes (7, 8, 9 10) de la deuxième surface d'articulation supérieure (2) sont dimensionnés pour être plus petits de 5 à 20 % que ceux des courbures correspondantes de la première surface principale d'articulation (1).

3. Articulation selon les revendications 1 ou 2, **caractérisée en ce que** les différences de valeur des rayons entre une face médiale (11) orientée vers le milieu du corps et une face latérale (12) orientée vers l'extérieur du corps différent les unes des autres pour des positions angulaires (V, H) concordantes de l'articulation (3).

4. Articulation selon au moins une des revendications précédentes, **caractérisée en ce que** les courbures (7, 8, 9, 10) des plans parallèles de la deuxième surface d'articulation principale (2) présentent des rayons qui diminuent dans la position angulaire (V) orientée vers le haut de la face latérale (12) vers la face médiale (11) de l'articulation (3).

5. Articulation selon au moins une des revendications précédentes, **caractérisée en ce que** les courbures (7, 8, 9, 10) des plans parallèles de la deuxième surface d'articulation (2) présentent des rayons qui diminuent dans la position angulaire (H) orientée vers le bas de la face médiale (11) vers la face latérale (12) de l'articulation (3).

6. Articulation selon au moins une des revendications précédentes, **caractérisée en ce que** les rayons des courbures (7, 8, 9, 10) sont dimensionnés pour être, sur la face latérale (12), plus petits dans la position angulaire (V) orientée vers le haut que dans la position angulaire (H) orientée vers le bas, et sur la face médiale (11) plus grands dans la position angulaire (V) orientée vers le haut que dans la position angulaire (H) orientée vers le bas.

7. Articulation selon au moins une des revendications précédentes, **caractérisée en ce que** les courbures (7, 8, 9 10) sont constituées de quelques rayons individuels présentant une transition continue.

8. Articulation selon au moins une des revendications précédentes, **caractérisée en ce que** la première surface d'articulation principale (1) et/ou la deuxième surface d'articulation principale (2) est réalisée transversalement au plan fonctionnel principal pour être plus large dans la position angulaire (V) orientée vers l'avant que dans la position angulaire (H) orientée vers le bas.

9. Articulation selon au moins une des revendications précédentes, **caractérisée en ce que** le rapport dimensionnel entre la première surface d'articulation principale (1) et la deuxième surface d'articulation principale (2) est d'approximativement 2/3.

10. Articulation selon au moins une des revendications précédentes, **caractérisée en ce que** la première surface d'articulation principale (1) et/ou la deuxième surface d'articulation principale (2) correspond à une découpe de surface d'un corps symétrique en rotation.

11. Articulation selon la revendication 10, **caractérisée en ce que** la découpe de surface correspond à un cylindre ou à une sphère.

12. Articulation selon la revendication 10, **caractérisée en ce que** la découpe de surface correspond à un hyperboloïde de révolution.

13. Articulation selon la revendication 10, **caractérisée en ce que** la découpe de surface passe de la convexité de la face médiale (11) à une concavité sur la deuxième surface d'articulation principale (2) en passant par une convexité de la face latérale (12) en une concavité en pente descendante raide qui débouche dans un pôle latéral.

14. Articulation selon au moins une des revendications précédentes, **caractérisée en ce que** la première surface d'articulation principale (1) correspondante présente une forme adaptée à celle de la deuxième surface d'articulation principale (2) avec une différence dimensionnelle minime.

15. Articulation selon au moins une des revendications précédentes, **caractérisée en ce que** la première surface d'articulation principale (1) est associée sous la forme d'une unité à la fois au tibia et à la fibule.

16. Articulation selon au moins une des revendications précédentes, **caractérisée en ce que** la première surface d'articulation principale (1) est constituée de deux éléments de structure.

17. Articulation selon la revendication 16, **caractérisée en ce que** les deux éléments de structure sont assemblés l'un à l'autre dans un plan parallèle au plan fonctionnel principal.
